(19) Europäisches Patentamt
European Patent Office
Office européen des brevets

(11) EP 2 865 443 A1

(12) EUROPEAN PATENT APPLICATION

(43) Date of publication:
**29.04.2015 Bulletin 2015/18**

(21) Application number: **13190091.2**

(22) Date of filing: **24.10.2013**

(51) Int Cl.:
***B01J 13/12*** *(2006.01)*
***B01J 13/14*** *(2006.01)*
***A23P 1/04*** *(2006.01)*
***A61K 9/50*** *(2006.01)*
***C09B 67/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Max-Planck-Gesellschaft zur Förderung der Wissenschaften e.V.**
  **80539 München (DE)**
- **University of Copenhagen**
  **1165 Copenhagen K (DK)**

(72) Inventors:
- **Landfester, Katharina, Prof.Dr.**
  **55122 Mainz (DE)**
- **Balouchev, Stanislav, Dr.**
  **55122 Mainz (DE)**
- **Risbo, Jens**
  **2720 Vanlose (DK)**
- **Hanner, Anna Justina**
  **217 53 Malmö (SE)**

(74) Representative: **Manitz, Finsterwald & Partner GbR**
**Martin-Greif-Strasse 1**
**80336 München (DE)**

(54) **Capsule, namely nanocapsule, microcapsule or macrocapsule, having a very low oxygen permeability**

(57) A capsule, namely a nanocapsule, microcapsule or macrocapsule, comprises a shell and a core, wherein the core contains at least one compound being at least at any temperature between more than 0°C and 80°C at 101,325 kPa a liquid, and wherein the shell comprises semicrystalline cellulose nanofibers and/or semicrystalline cellulose nanocrystals having each a crystallinity index of at least 30%. These capsules have in particular a very low oxygen permeability and thus protects the liquid included in its core from oxygen. Accordingly, these capsules may be used for protecting for example food or food ingredients from oxygen. In addition, these capsules may be used to protect photoactive compounds from oxygen, such as phosphorescent compounds and photon upconversion compositions, in particular triplet-triplet annihilation upconversion compositions.

EP 2 865 443 A1

## Description

The present invention relates to a capsule and in particular to a nanocapsule, microcapsule or macrocapsule, enclosing a liquid, to a method for producing such a capsule and to the use of such a capsule.

Nano-, micro- and macrocapsules enclosing a liquid may be used for a plurality of applications, such as for the delivery of a liquid - which includes for example a therapeutic molecule - to its target, such as for example to a specific tissue or the like. Moreover, such capsules may be used to protect the enclosed liquid against the environment, such as against gases, against radicals, against chemical compounds and against physical evaporation, e.g. against ozone, oxygen, prooxidants, radicals, peroxides and the like and in particular against molecular oxygen. The liquid may be in particular a drug, a food or a food ingredient being sensitive to chemical degradation initiated by the presence of molecular oxygen, such as a fat or oil.

One further promising field for such nano-, micro- and macrocapsules is the protection of photoactive compositions, such as phosphorescent compositions or photon upconversion compositions, from molecular oxygen. Such photoactive compositions are used in a plurality of applications, such as in organic light-emitting devices, solar cells, inks, thermal sensors, oxygen sensors, coating materials and the like. Both phenomena, phosphorescence and photon upconversion involve the excitation of triplet states in the concerned molecules after absorption of light and are characterized by the emission of light. While phosphorescent materials emit light having a lower frequency than that of the absorbed light, photon upconversion compositions emit light with a higher frequency than that of the absorbed light. The latter mentioned phenomenon is often associated with high light intensities, such as those available from pulsed lasers. The upconversion process involves the energy transfer of several lower excited states to a single higher excited state, which then emits light with a higher frequency, i.e. with higher energy. Typical photon upconversion compositions comprise a sensitizer compound, an emissive compound and a solvent or matrix compound, respectively. One prominent example for such photon upconversion systems is a triplet-triplet annihilation upconversion system (TTA-UC). Due to their ability to convert that part of the sunlight with comparatively long wavelengths to light having a shorter wavelength, TTA-UC compositions are particular promising materials for solar cells. Apart from that, TTA-UC compositions may be also used for high-resolution optical microscopy, solar harvesting, bioimaging, drug delivery, optical data storage, oxygen sensing and the like. As set out above, the TTA-UC compositions may include the sensitizer compound and emissive compound dispersed in a solvent or polymer matrix. However, such photoactive compositions and in particular phosphorescence and TTA-UC compositions are very sensitive to oxygen. This is due to the fact that oxygen is an effective quencher of excited triplet states.

In order to overcome these problems, phosphorescent and TTA-UC compositions are usually degassed for example by bubbling molecular nitrogen through the solution or around the solid, in order to remove at least a part of the oxygen contained therein or in the surrounding atmosphere. These methods are, however, not satisfying, because they do not allow to remove all of the oxygen present in the composition or in the surrounding atmosphere. Moreover, at least some of these methods are time consuming and laborious. In addition, these methods do not avoid that during the use of the compositions again oxygen diffuses from the environment into the composition.

Due to this, it has been already proposed to enclose such photoactive compositions in nano- or microcapsules as passive oxygen protection. For example, Kim and Kim describe in "Encapsulated triplet-triplet annihilation-based upconversion in the aqueous phase for sub-band-gap semiconductor photocatalysis "in the Journal of the American Chemical Society, 2012, 134, pages 17478 to 17481 a TTA-UC composition being encapsulated in a microcapsule having a shell made of ethoxylated trimethylolpropane triacylate ester (ETPTA). Moreover, Liu et al. describe in "A general strategy for biocompatible, high-effective upconversion nanocapsules based on triplet-triplet annihilation" in the Journal of the American Chemical Society, 2013, 135, pages 5029 to 5037 nanocapsules in the form of bovine serum albumin-dextran stabilized oil droplets enclosing a TTA-UC composition, in order to protect it among others against molecular oxygen. However, these nano- and microcapsules are not completely satisfying, because their oxygen permeability is still quite high. Due to this, even if protecting its content against molecular oxygen for a while, the photoactive compositions enclosed therein start to get quenched after a comparatively short period of time.

Accordingly, the object underlying the present invention is to provide a capsule and particularly a nanocapsule, microcapsule or macrocapsule having an improved impermeability against gases, radicals, chemical compounds and particularly against molecular oxygen as well as an excellent mechanical stability, so that it can efficiently protect liquid enclosed therein, such as a food, a food ingredient or a photoactive composition, such as in particular a phosphorescent or TTA-UC composition, in particular against molecular oxygen for an increased period of time, which therefore can be used in ambient atmosphere.

According to the present invention this object is satisfied by providing a capsule, particularly a nanocapsule, microcapsule or macrocapsule, comprising a shell and a core, wherein the core contains at least one compound being at least at any temperature between more than 0°C and 80°C at 101,325 kPa a liquid, and wherein the shell comprises semicrystalline cellulose nanofibers and/or semicrystalline cellulose nanocrystals having each a crystallinity index measured by X-ray diffraction of at least 30%.

This solution is based on the surprising finding that by providing specific cellulose nanofibers and/or cellulose nanocrystals, namely cellulose nanofibers having a crystallinity index of at least 30% and/or cellulose nanocrystals having a crystallinity index of at least 30%, into the shell of a capsule, a nanocapsule, microcapsule or macrocapsule, respectively, is obtained, which has a very high impermeability against components which may be present in the environment or in the core of the capsule, namely gases, radicals, chemical compounds and the like and in particular against molecular oxygen, as well as an excellent mechanical stability, why it is excellently well suited to enclose and protect liquids being sensitive to oxygen, i.e. it function as passive oxygen protection, or to other gases and/or liquid compounds and/or solid compounds. More specifically, the oxygen permeability of these nanocapsules, microcapsules and macrocapsules is two orders of magnitude lower than that of the best known petrochemical alternatives, such as ethylene-vinyl alcohol-copolymer. While the high mechanical strength of the respective nanocapsules, microcapsules and macrocapsules is due to fiber reinforcement on account of the presence of the cellulose nanofibers and/or cellulose nanocrystals, the excellent oxygen impermeability of these nanocapsules, microcapsules and macrocapsules is a result of the semicrystallinity of the cellulose nanofibers and/or cellulose nanocrystals. Due to the semicrystallinity of the cellulose nanofibers and cellulose nanocrystals, these fibers and crystals contain crystalline regions or domains being virtually impermeable against molecular oxygen. These crystalline regions or crystallites are connected with each other by amorphous regions or less ordered regions giving the cellulose nanofibers enough flexibility along the length of the nanofibers so that they can be prepared within the shell of nanocapsule, microcapsule or macrocapsule irrespective of their shape and size. In other words, the amorphous regions or less ordered regions of the cellulose nanofibers act as flexible joints along the length of the nanofibers connecting the crystalline regions of the nanofibers which are responsible for the high impermeability of the nanofibers against molecular oxygen. On account of this, the size of the individual crystalline regions of the nanofibers and nanocrystals is not essential, but in fact the crystallinity index. Due to all these reasons, the nano-, micro- and macrocapsules in accordance with the present invention are perfectly well suited to encapsulate liquid being sensitive against gases and in particular against molecular oxygen, such as pharmaceutical compositions, foodstuff, volatile flavor and photoactive compositions, such as phosphorescent and TTA-UC compositions. Moreover, these nano-, micro- and macrocapsules are perfectly well suited to encapsulate liquid in order to protect the liquid against radicals, chemical compounds and physical evaporation, such as against prooxidants, radicals and/or peroxides. A further advantage of the nanocapsules, microcapsules and macrocapsules according to the present invention is that they are comparatively cheap, because the applied cellulose fibers are cheap and because they can be manufactured with easy and cost efficient methods.

A further advantage of the present patent application is that it enables the transformation of a liquid - e.g. a pure liquid or a liquid containing a component of interest - into a powder, namely a powder comprising - among other components - the capsules according to the present invention. Such a powder state has several benefits compared to the liquid state, such as it allows a convenient dosing and handling, a high storage stability, a slow uptake of oxygen and a slow loss of volatile components. This is specifically of interest in cases where the liquid is a purely or partly volatile aroma component. This embodiment is particularly suitable for the field of food and pharmaceutical production.

In accordance with the present invention, a nanocapsule is any container having a regular or irregular form, wherein the longest dimension of the container is between 1.00 and 999.99 nm. Likewise to this, a microcapsule is defined in accordance with the present invention as any container having a regular or irregular form, wherein the longest dimension of the container is between 1.00 and 999.99 μm. Likewise to this, a macrocapsule is defined in accordance with the present invention as any container having a regular or irregular form, wherein the longest dimension of the container is at least 1 mm and preferably between 1 and 100 mm. Moreover, in accordance with the present invention the terms "capsule" and "container" denote any article having a cavity, irrespective of the outer form of the article.

Furthermore, a nanofiber is defined in accordance with the present invention as any fiber having a diameter between 1.00 and 999.99 nm or a length between 1 and 5,000 nm, wherein the aspect ratio is at least 2. Also a nanocrystal is defined in accordance with the present invention as any structure having a diameter between 1.00 and 999.99 nm or a length between 1 and 2,000 nm, wherein the aspect ratio is at least 2. The difference between a cellulose nanofiber and a cellulose nanocrystal is in accordance with the present invention that a nanofiber can be further degraded by acid hydrolysis meaning that the length of the nanofiber reduces during an acid hydrolysis, whereas a nanocrystal is essentially not degraded by an acid hydrolysis and thus essentially maintains its length during an acid hydrolysis. More specifically, a cellulose nanostructure having an aspect ratio of at least 2 is a nanofiber in accordance with the present invention, when the ratio of the length of the cellulose nanostructure after an acid hydrolysis divided by the length of the cellulose nanostructure before the acid hydrolysis is at most 0.5, when the acid hydrolysis is conducted for 30 minutes at 65°C to 70°C with continuous stirring in suspension containing a 64 % by weight aqueous sulfuric acid solution and 4 % by weight cellulose nanostructure material. In contrast to this, a nanocrystal is a cellulose nanostructure having an aspect ratio of at least 2, wherein the ratio of the length of the cellulose nanostructure after the acid hydrolysis performed at the aforementioned conditions divided by the length of the cellulose nanostructure before the acid hydrolysis is more than 0.5.

The crystallinity index is measured in accordance with the present invention by X-ray diffraction. More specifically, the crystallinity index of the semicrystalline cellulose nanofibers and/or cellulose nanocrystals is determined according to the method described as the peak height method in Park et al. "Cellulose crystallinity index: measurement techniques and their impact on interpreting cellulose performance", Biotechnology for Biofuels 2010, 3:10 making reference to Segal et al. "An empirical method for estimating the degree of crystallinity of native cellulose using the x-ray diffractometer" in Text Res J, 1959, pages 786 to 794. Accordingly, the crystallinity index is calculated from the X-ray diffractogram (XRD profile) obtained for a sample of a cellulose nanofiber or a cellulose nanocrystal, such as freeze dried or solvent-cast films of nanofibers or nanocrystals, according to the following equation:

$$CI = 100 \times (I_{002} - I_{am}) / I_{002},$$

wherein CI is the crystallinity index, $I_{002}$ is the peak intensity at plane (002) in the XRD profile and $I_{am}$ is the intensity at $2\theta = 18°$, which is the reported angle at which the maximum intensity for a totally amorphous sample is typically observed when using a Cu x-ray source. If another source is used, the angle has to be recalculated accordingly. The relevant intensities are depicted in figure 1 a of Park et al. (2010). It should be noted that the sample should have a thickness and density high enough to give a significant x-ray intensity as compared to any background signal arising from the apparatus and sample holder. Small background contributions can corrected, as indicated in figure 1a of Park et al., with the background being determined by linear interpolation of the intensity outside the angle of interest.

In tendency, the oxygen impermeability of the nano-, micro- and macrocapsules in accordance with the present invention increases with increased crystallinity index of the semicrystalline cellulose nanofibers and/or cellulose nanocrystals, respectively. However, a certain amount of amorphous or less ordered regions is required so that the nanofibers are flexible enough and therefore processable. In view of this, the shell of the capsule preferably comprises semicrystalline cellulose nanofibers and/or cellulose nanocrystals having a crystallinity index of at least 40%, more preferably at least 50%, even more preferably at least 55%, still more preferably of at least 60% and most preferably of at least 70%. Moreover, the shell of the capsule preferably comprises semicrystalline cellulose nanofibers and/or cellulose nanocrystals having a crystallinity index of at most 99.9%, more preferably at most 95%, even more preferably at most 90%, still more preferably of at most 85% and most preferably of at most 80%. Accordingly, the shell of the capsule preferably comprises semicrystalline cellulose nanofibers and/or cellulose nanocrystals having a crystallinity index of between 30 and 99.9%, more preferably between 40 and 99.9%, even more preferably between 50 and 95%, still more preferably between 60 an 90% and most preferably between 70 and 80%, such as for example about 60% or 70%.

The present invention is not specifically restricted with regard to the shape and size of the nano-, micro- or macrocapsule. However, in view of the processability, a sufficiently thick shell wall and the required, but not extensively more than sufficient volume for encapsulating the liquid, the longest dimension of the shell of the capsule is preferably at most 1 cm, more preferably between 0.1 and 5 μm, even more preferably between 0.6 and 4 μm and most preferably between 1 and 2 μm, such as for example between 1.2 and 1.5 μm.

In accordance with the present invention the term "longest dimension" of the shell of the capsule is defined as the longest distance between two points on the outer surface of the shell connected via a line. For example, the "longest dimension" of a hollow sphere is the diameter of the hollow sphere, whereas the "longest dimension" of a hollow ellipsoid is the longest of all axes of the ellipsoid.

It has been found in the present invention that the degree of oxygen permeability is strongly influenced by the thickness of the shell wall of the capsule, respectively. In tendency, the higher the shell wall thickness, the lower the oxygen permeability of the capsule. In view of this the shell wall has according to a preferred embodiment of the present invention an average thickness of between 1 nm and 50 μm, more preferably between 5 and 100 nm or between more than 5 nm and 25 μm, even more preferably between 10 and 500 nm and most preferably between 20 and 30 nm.

In accordance with the present invention the "average thickness" of the shell wall of the capsule is the arithmetic mean of the shell wall thicknesses at all locations of the shell. This is measured by cutting through a capsule, selecting 20 arbitrary locations at the cut surface of the shell wall and then measuring the thickness at any of these locations by means of a scanning electron microscope or transmission electron microscopy. Afterwards, the arithmetic mean from the 20 individual values is calculated.

Moreover, it has been found in the present invention that - at a given average thickness of the shell wall of the capsule - a better oxygen impermeability is obtained, if the thickness of the shell wall is as homogenous as possible, i.e. the lower the variation of the individual shell wall thickness values at different locations of the shell is. Preferably, at least 70% of all shell wall thickness values at different locations of the shell lie in a range between 50 and 200%, more preferably between 75 and 150% and most preferably between 90 and 110% of the average thickness of the shell wall. More preferably, the aforementioned ranges are fulfilled by at least 80% of all shell wall thickness values, even more preferably by at least 90%, particularly preferably by at least 95% and most preferably by all of the shell wall thickness values. The respective values are determined by cutting through a capsule, selecting 20 arbitrary locations at the cut

surface of the shell wall, then measuring the thickness at any of these locations by means of a scanning electron microscope or transmission electron microscopy and thereafter calculating the respective values.

In addition, it has been found in the present invention that the degree of oxygen permeability may be strongly influenced by the water content of the shell of the capsule. In tendency, the lower the water content of the shell, the lower the oxygen permeability of the capsule. However, the dependency of the oxygen permeability from the water content can be significantly reduced by crosslinking the fibers and/or crystals with each other as described further below with regard to a particular preferred embodiment of the present invention.

In view of this, the shell of the capsule in accordance with the present invention preferably has a water content of at most 40% by weight, more preferably of at most 10% by weight and most preferably of at most 5% by weight. The appropriate water content of the shell of the capsule can be easily adjusted by drying the capsule after its preparation for a suitable period of time at appropriate conditions.

In accordance with the present invention the "water content" of the shell of the capsule is measured gravimetrically.

According to a further preferred embodiment of the present invention, comparatively short semicrystalline cellulose nanofibers are provided in the shell of the nanocapsule, microcapsule or macrocapsule. More specifically, the cellulose nanofibers preferably have an average length of between 1 and 5,000 nm, more preferably between 1 and 1,000 nm, even more preferably between 50 and 1,000 nm, even more preferably between 150 and 600 nm and most preferably between 250 and 500 nm. In particular, such comparable short fibers in combination with cellulose nanocrystals are used, since they form a closely packed and dense structure. If the capsule comprises instead of semicrystalline cellulose nanofibers or in addition to semicrystalline cellulose nanofibers semicrystalline cellulose nanocrystals, the cellulose nanocrystals preferably have an average length of between 40 and 2,000 nm, more preferably between 60 and 2,000 nm, even more preferably between 80 and 2,000 nm and most preferably between more than 100 and 2,000 nm.

Similarly to the average thickness of the shell wall of the capsule, the average length of the cellulose nanofibers and cellulose nanocrystals is defined in accordance with the present invention as the arithmetic mean of the lengths of all cellulose nanofibers and cellulose nanocrystals. This is measured by selecting at least 200 arbitrary fibers or crystals and then measuring the length of each of the fibers or crystals by means of a scanning electron microscope or transmission electron microscopy. Afterwards, the arithmetic mean from the at least 200 individual values is calculated.

Preferably, the lengths of the individual cellulose nanofibers and/or cellulose nanocrystals are as homogenous as possible, i.e. the individual cellulose nanofibers and/or cellulose nanocrystals have only a low variation in their length. Preferably, at least 50% of all cellulose nanofibers and/or cellulose nanocrystals of the shell have a length lying in a range between 10 and 400%, more preferably between 20 and 200% and most preferably between 40 and 150% of the average length of the cellulose nanofibers and/or cellulose nanocrystals. More preferably, the aforementioned ranges are fulfilled by at least 60%, even more preferably by at least 70%, particularly preferably by at least 90% and most preferably by all of the cellulose nanofibers and/or cellulose nanocrystals. The respective values are determined by selecting at least 200 arbitrary cellulose nanofibers or cellulose nanocrystals, then measuring the length of any cellulose nanofiber or cellulose nanocrystals by means of a scanning electron microscope and thereafter calculating the respective values.

In principle, the present invention is not particularly limited with regard to the diameter of the cellulose nanofibers. However, good results are achieved if the cellulose nanofibers have an average diameter of between 1 and 100 nm, more preferably between 2 and 40 nm, even more preferably between 4 and 10 nm and most preferably between 5 and 7 nm.

Similarly to the average length of the cellulose nanofibers, the average diameter of the cellulose nanofibers is defined in accordance with the present invention as the arithmetic mean of the diameters of all cellulose nanofibers. This is measured by selecting at least 200 arbitrary fibers and then measuring the diameter of each of the fibers by means of a scanning electron microscope or transmission electron microscopy or atomic force microscopy. Afterwards, the arithmetic mean from the at least 200 individual values is calculated.

Preferably, the diameters of the individual cellulose nanofibers are as homogenous as possible, i.e. the individual cellulose nanofibers have only a low variation in their diameter. Preferably, at least 70% of all cellulose nanofibers of the shell have a diameter lying in a range between 20 and 200%, more preferably between 40 and 150% and most preferably between 80 and 120% of the average diameter of the cellulose nanofibers. More preferably, the aforementioned ranges are fulfilled by at least 80%, even more preferably by at least 90%, particularly preferably by at least 95% and most preferably by all of the cellulose nanofibers. The respective values are determined by selecting at least 200 arbitrary cellulose nanofibers, then measuring the diameter of any cellulose nanofiber by means of a scanning electron microscope or transmission electron microscopy or atomic force microscopy and thereafter calculating the respective values.

If the capsule comprises instead of semicrystalline cellulose nanofibers or in addition to semicrystalline cellulose nanofibers semicrystalline cellulose nanocrystals, the cellulose nanocrystals preferably have an average diameter of between 1 and 100 nm, more preferably between 2 and 40 nm, even more preferably between 4 and 10 nm and most preferably between 5 and 7 nm. The average diameter of the cellulose nanocrystals is defined in accordance with the

present invention as the arithmetic mean of the diameter of all cellulose nanocrystals. This is measured by selecting at least 200 arbitrary crystals and then measuring the diameter of each of the crystals by means of a scanning electron microscope or transmission electron microscopy or atomic force microscopy. Afterwards, the arithmetic means from the at least 200 individual values is calculated.

In order to achieve a particularly high reinforcement effect and thus a nanocapsule, microcapsule or macrocapsule having a particularly high mechanical stability, it is suggested in accordance with a further preferred embodiment of the present invention that cellulose nanofibers and/or cellulose nanocrystals having a sufficiently high aspect ratio are provided in the shell of the capsule, because it has been found in the present invention that cellulose nanofibers and cellulose nanocrystals having a small aspect ratio only lead to a comparatively low mechanical stability of the capsule. In view of this, it is proposed that the cellulose nanofibers and/or the ellulose nanocrystals of the capsule have an average aspect ratio of preferably between 5 and 1,000, more preferably between 10 and 150, even more preferably between 30 and 100 and most preferably between 50 and 80, such as for example about 70.

In accordance with the present invention, the average aspect ratio is calculated from at least 200 aspect ratios. The aspect ratio is the length of a cellulose nanofiber or a cellulose nanocrystal divided by its diameter.

Preferably, also the aspect ratios of the individual cellulose nanofibers and/or the individual cellulose nanocrystals are as homogenous as possible, i.e. the individual cellulose nanofibers and/or the individual cellulose nanocrystals have only a low variation in their aspect ratio. Preferably, at least 70% of all cellulose nanofibers and/or of all cellulose nanocrystals of the shell have an the aspect ratio lying in a range between 20 and 200%, more preferably between 40 and 150% and most preferably between 80 and 120% of the average the aspect ratio of the cellulose nanofibers and/or cellulose nanocrystals. More preferably, the aforementioned ranges are fulfilled by at least 80%, even more preferably by at least 90%, particularly preferably by at least 95% and most preferably by all of the cellulose nanofibers and/or cellulose nanocrystals. The respective values are determined by selecting at least 200 arbitrary cellulose nanofibers and/or cellulose nanocrystals, then measuring the length and diameter of any cellulose nanofiber and/or cellulose nanocrystal by means of a scanning electron microscope or transmission electron microscopy or atomic force microscopy, then calculating the aspect ratio of any cellulose nanofiber or cellulose nanocrystal by dividing the length by the diameter and thereafter calculating the arithmetic mean from the respective aspect ratio values.

According to the present invention, the shell of the capsule comprises either i) semicrystalline cellulose nanofibers having a crystallinity index of at least 30 or ii) semicrystalline cellulose nanocrystals having a crystallinity index of at least 30 or iii) a blend of semicrystalline cellulose nanofibers and of cellulose nanocrystals having each a crystallinity index of at least 30. Preferably, the shell of the capsule comprises semicrystalline cellulose nanofibers having a crystallinity index of at least 30 alone or in admixture with semicrystalline cellulose nanocrystals having a crystallinity index of at least 30. Preferably, the weight ratio of semicrystalline cellulose nanofibers to semicrystalline cellulose nanocrystals is 100:0 to 10:90, more preferably 99:1 to 40:60 and most preferably 90:10 to 60:40.

Usually, good results are achieved when unmodified semicrystalline cellulose nanofibers and/or cellulose nanocrystals are provided in the shell of the nanocapsule, microcapsule or macrocapsule. However, in certain cases it is advantageous that modified semicrystalline cellulose nanofibers and/or cellulose nanocrystals are provided in the shell of the capsule, for example in order to adjust the hydrophobicity or hydrophilicity of the inner or outer surface of the shell of the capsule, respectively, in order to provide a basis for a crosslinking of the nanofibers and/or cellulose nanocrystals. In view of this, it is possible and in certain embodiments preferred that at least a part or even all of the semicrystalline cellulose nanofibers and/or cellulose nanocrystals of the shell of the capsule are surface modified with a functional group selected from the group consisting of isocyanate groups, hydroxyl groups, carboxylic acid groups, amino groups, thiol groups, epoxy groups, acrylate groups, alkyne groups, azide groups and arbitrary combinations of two or more of the aforementioned groups. Alternatively or in addition thereto, at least a part or even all of the semicrystalline cellulose nanofibers and/or cellulose nanocrystals of the shell of the capsule may be surface modified with a polymer, oligomer or monomer, wherein the polymer, oligomer or monomer may comprise a functional group selected from the group consisting of isocyanate groups, hydroxyl groups, carboxylic acid groups, amino groups, thiol groups, epoxy groups, acrylate groups, alkyne groups, azide groups and arbitrary combinations of two or more of the aforementioned groups. For instance, the polymer, oligomer or monomer may be grafted on the cellulose nanofibers or may be physically absorbed thereon.

In order to obtain a nanocapsule, microcapsule or macrocapsule having a particular excellent mechanical stability, it is suggested according to a further particularly preferred embodiment of the present invention that the semicrystalline cellulose nanofibers and/or cellulose nanocrystals of the shell of the nanocapsule, microcapsule or macrocapsule are present in the form of a three-dimensional network, in which at least some of the semicrystalline cellulose nanofibers and/or cellulose nanocrystals are connected with one or more other semicrystalline cellulose nanofibers and/or cellulose nanocrystals of the shell of the capsule. In principle, at least some of the semicrystalline cellulose nanofibers and/or cellulose nanocrystals may be directly connected with one or more other semicrystalline cellulose nanofibers and/or cellulose nanocrystals of the shell of the capsule by any kind of bond. Alternatively or in addition thereto, at least some of the semicrystalline cellulose nanofibers and/or cellulose nanocrystals may be indirectly connected with one or more other semicrystalline cellulose nanofibers and/or cellulose nanocrystals of the shell of the capsule by means of a further compound acting as "joint".

Concerning the kind of connection between the individual cellulose nanofibers and/or cellulose nanocrystals forming the network the present invention is not particularly limited. Thus, the individual cellulose nanofibers and/or cellulose nanocrystals of this particularly preferred embodiment may be connected with each other by means of any known intramolecular force and/or any known intermolecular force.

In order to achieve a particular firm connection between the individual fibers and/or crystals, the semicrystalline cellulose nanofibers and/or cellulose nanocrystals of the capsule in accordance with this embodiment may be connected with each other by covalent bonds. This is preferably achieved by crosslinking the cellulose nanofibers and/or cellulose nanocrystals with a crosslinking group.

There is virtually no limitation about respective crosslinking groups provided that they can be linked to unmodified or modified cellulose molecules. For instance, any crosslinking group selected from the group consisting of non-substituted or substituted alkyl groups, alkenyl groups, alkynyl groups, alicyclic groups, aralkyl groups, aromatic groups, heterocyclic groups and arbitrary combinations of two or more of the aforementioned groups may be used for this purpose, wherein the crosslinking group may be for example connected with the cellulose nanofibers and/or cellulose nanocrystals by means of any group selected from the group consisting of acrylate groups, methacrylate groups, urethane groups, urea groups and arbitrary combinations of two or more of the aforementioned groups. Such a crosslinking of the cellulose nanofibers and/or cellulose nanocrystals may be achieved e.g. by reacting a bifunctional crosslinking group with cellulose nanofibers and/or cellulose nanocrystals being modified with one or more group(s) being reactive with the functional groups of the crosslinking group. For instance, unmodified cellulose nanofibers and/or cellulose nanocrystals may be reacted with a diisocyanate compound under formation of crosslinked cellulose nanofibers and/or cellulose nanocrystals. Alternatively, the cellulose nanofibers and/or cellulose nanocrystals may be first surface modified for instance by grafting isocyanate groups thereon and then the modified cellulose nanofibers and/or cellulose nanocrystals may be reacted with a diisocyanate compound under formation of crosslinked cellulose nanofibers and/or cellulose nanocrystals. While in the first case the crosslinking groups and cellulose molecules are connected via an urethane bond, in the latter case the crosslinking groups and cellulose molecules are connected via an urea bond.

Alternatively, the semicrystalline cellulose nanofibers and/or cellulose nanocrystals of the nanocapsule, microcapsule or macrocapsule in accordance with this particularly preferred embodiment may be connected with each other by ionic bonds, in order to achieve a particular firm connection between the individual fibers and/or crystals and thus a strong network of cellulose fibers and/or crystals. This may be for example achieved by reacting cationic or anionic cellulose nanofibers and/or cellulose nanocrystals with a compound selected from the group consisting of polyelectrolytes, multivalent molecules, monomer and oligomers, or, alternatively, by a compound selected from the group consisting of polyelectrolytes, multivalent molecules, monomer and oligomers, acting as intermediates between cationic or anionic cellulose nanofibers, such as for example charged polysaccharides, charged oligomers, charged molecules, ions (e.g. calcium), chitosan, pectine, alginate, proteins, amino acids, polypeptides, sodium carboxymethylated cellulose, cellulose derivatives, modified starch and derivatives of the previously mentioned molecules. Of course it is also possible, to combine the two aforementioned variants, i.e. to provide cellulose nanofibers and/or cellulose nanocrystals in the shell of the capsule, which are connected with each other by ionic bonds as well as by covalent bonds.

Alternatively, the semicrystalline cellulose nanofibers and/or cellulose nanocrystals of the capsule in accordance with this particularly preferred embodiment may be connected with each other by physical bonds. In accordance with the present invention, the term "physical bond" denotes any intermolecular force, such as, but not limited to dipole-dipole interactions (such as hydrogen bonds), ion-dipole interactions, van der Waals interactions and the like. In contrast to this, covalent bonds and ionic bonds are referred to as intramolecular forces. Such a physical bond may bond two or more semicrystalline nanofibers and/or cellulose nanocrystals directly with each other. Alternatively, such a physical bond may be for example achieved by providing in the shell of the capsule - in addition to cellulose nanofibers and/or cellulose nanocrystals - a hemicellulose, e.g. xylan, wherein the xylan will irreversibly adsorb onto the cellulose nanofiber surface and/or cellulose nanocrystals, thus crosslinking the cellulose nanofibers and/or cellulose nanocrystals. Instead of xylan, also glucuronoxylan, arabinoxylan and/or glucomannan may be used. Moreover, such a physical bond may be accomplished by an adsorption mechanism or by a solidification mechanism, namely for example by a solvent evaporation or a precipitation mechanism as a consequence of temperature, pH change, addition of non-solvent or salt during the preparation of the capsules. Of course it is also possible, to combine the aforementioned variants, i.e. to provide cellulose nanofibers and/or cellulose nanocrystals in the shell of the capsule, which are connected with each other by physical bonds and/or covalent bonds and/or ionic bonds.

According to a further particular preferred embodiment of the present invention, the shell of the capsule does not only consist of the crosslinked or non-crosslinked semicrystalline cellulose nanofibers and/or cellulose nanocrystals, but further comprise an oligomer and/or a polymer and/or other compounds and/or ions, such as sugars, amino acids, calcium ions, which - depending on the amount - either form the matrix of the shell, into which the cellulose nanofibers and/or cellulose nanocrystals are embedded, or fill small gaps between the cellulose nanofibers and/or cellulose nanocrystals. In addition, it is possible that the shell of the capsule is a two-layer or higher multiple-layer structure. In the case of a two-layer or a higher multiple-layer the inner layer is preferably mainly composed of the oligomer and/or polymer, wherein the outer layer is preferably composed of a mixture of cellulose nanofibers and/or cellulose nanocrystals and oligomer and/or polymer with the cellulose nanofibers being the main component of the outer layer.

The present embodiment is not particularly limited concerning the chemical nature of the oligomer and/or polymer and/or other compound and/or ions, such as sugars, amino acids and/or calcium ions. Suitable examples therefor are polymers selected from the group consisting of polyureas, polyurethanes, polyacrylates, polymethacrylates, polyethers, polyamides and arbitrary combinations of two or more of the aforementioned polymers. Particularly good results are achieved with polyureas and polyurethanes.

Preferably, the weight ratio of i) the semicrystalline cellulose nanofibers and/or cellulose nanocrystals to ii) polymer in the shell amounts to 100:0.1 to 0.1:99.9, preferably 80:20 to 10:90 and most preferably 70:30 to 14:86.

In principle, the cellulose nanofibers may be prepared from any cellulose material of natural origin or from synthetic cellulose. However, good results are in particular obtained with nanofibers being prepared from cellulose of natural origin. For example, the semicrystalline cellulose nanofibers may be prepared from plant cellulose, tunicin cellulose and/or bacterial cellulose e.g. by chemical modification, acid hydrolysis, enzymatic treatment and/or mechanical disintegration.

Moreover, the cellulose nanocrystals may be prepared from cellulose nanofibers by acid hydrolysis, which is the main extraction process, which degrades the less ordered parts of the nanofibers leaving the crystallites more or less intact. Cellulose nanocrystals are shorter than the cellulose nanofiber, from which they are produced.

According to a further variant of the present invention, the shell comprising the semicrystalline cellulose nanofibers and/or cellulose nanocrystals having each a crystallinity index of at least 30% is coated on its outer side with a coating layer or with two or more coating layers using for example the layer-by-layer technique. The coating layer may be provided to even further increase the oxygen impermeability and/or the mechanical strength of the capsule.

For example, the coating layer may be composed of a polysaccharide or a hydrophobic polymer, such as a wax. Preferred examples therefor are uncharged polysaccharides, such as cellulose or hemicelluloses, or charged polymers, such as chitosan, pectine, alginate, proteins, amino acids, polypeptides, sodium carboxymethylated cellulose, cellulose derivatives, cellulose nanofibers, modified starch and derivatives of the aforementioned molecules. The use of charged polymers allows the coating layer to absorb on the surface of the shell by means of electrostatic attractive forces.

According to a further preferred embodiment of the present invention, the shell of capsule has an elastic modulus of at least 1 GPa, preferably of at least 2 GPa, more preferably of at least 3 GPa, even more preferably of at least 4.5 GPa, still more preferably of at least 10 GPa, still more preferably of at least 20 GPa and most preferably of at least 138 GPa. If the shell is coated, the aforementioned values refer to the shell including the coating. The elastic modulus of the shell is measured in accordance with the present invention as described in A. Fery, R. Weinkamer, Polymer 2007, vol. 48, pages 7221 to 7235 and in H. J. Butt, B. Cappella, M. Kappl, Surf Sci Rep 2005, vol. 59, pages 1 to 152.

Concerning the chemical nature of the liquid enclosed by the shell of the nanocapsule, microcapsule or macrocapsule, the present invention is not particularly limited. According to the present invention, in this connection a liquid is a compound, which is at least at any temperature between more than 0°C and 80°C at 101,325 kPa in liquid form. Preferably, the liquid is at least at any temperature between more than 0°C and 50°C at 101,325 kPa, more preferably at least at any temperature between 10°C and 40°C at 101,325 kPa and even more preferably at least at 25°C at 101,325 kPa in liquid form.

In particular, any hydrophilic and any hydrophobic liquid may be encapsulated by the shell. In particular, the capsule in accordance with the present invention is suitable to enclose hydrophobic liquid(s). In accordance with the present invention, a hydrophobic liquid is a liquid that is not fully miscible with water or any other polar phase in which the cellulose nanofibers and/or the nanocrystals are dispersed, so that a two phase system is formed at a specific temperature, pressure and liquid composition.

In principle, the shell may contain the target compound alone or in admixture with a solvent or a dispersion agent.

For example, the core of the capsule may contain as target compound any compound selected from the group consisting of fats, oils, colloids, micelles, dyes, drugs, food ingredients, and arbitrary combinations of two or more of the aforementioned compounds.

According to a further particular preferred embodiment of the present invention, the target compound is at least one compound, which has a triplet state capable of energy transfer via an emissive process or a non-emissive process. Such compounds are particularly sensitive to oxygen, because oxygen is an effective quencher of excited triplet states. Preferably, the target compound is at least one phosphorescent compound or a TTA-UC composition, such as a mixture of at least one sensitizer compound being capable of absorbing radiation at a first frequency $v_1$ and of at least one emissive compound, wherein the at least one sensitizer compound is capable of transferring energy to the at least one emissive compound and wherein the at least one emissive compound, after obtaining energy transferred from the at least one sensitizer compound, is capable of emitting light at a second frequency $v_2$, wherein the following equation is

fulfilled: $v_2 > v_1$, wherein the at least one sensitizer compound is capable of a triplet-triplet energy transfer to the at least one emissive compound and wherein the at least one emissive compound is capable of a triplet-triplet annihilation

As set out above, the target compound may be included within the shell of the capsule alone or in admixture with at least one solvent and/ or with at least one dispersion agent.

There is virtually no limitation with regard to the chemical nature of the solvent and dispersion agent, respectively, provided that it is able to dissolve or disperse the target compound. For example, the solvent and/or dispersion agent may be selected from the group consisting of alkanes, alkenes, alkynes, aromatics, heterocyclics, ketones, aldehydes, sulfoxides, triglycerides, alcohols, water and arbitrary combinations of two or more of the aforementioned compounds. More specifically, $C_{6-20}$-alkanes, such as hexadecane, acetone, benzene, xylene, toluene, cyclohexane, dimethylformamide, ethanol, methanol and water are suitable solvents and dispersion agents, respectively.

Moreover, the present invention relates to a method for preparing the aforementioned capsule and in particular nanocapsule, microcapsule or macrocapsule. The method comprises the steps of:

a) providing a polar phase containing semicrystalline cellulose nanofibers and/or cellulose nanocrystals having each a crystallinity index of at least 30%,
b) providing a second phase containing i) at least one solvent and/or dispersion agent being immiscible with the polar phase obtained in step a), ii) at least one compound selected from the group consisting of monomers, oligomers, polymers and arbitrary combinations thereof and optionally iii) at least one target compound to be encapsulated,
c) dispersing the second phase obtained in step b) in the polar phase obtained in step a) and
d) incubating the dispersion obtained in step c).

Preferably, the polar phase provided in step a) is a mixture of a polar solvent or dispersion agent, such as water, a $C_{1-6}$-alcohol, dimethyl sulfoxides, dimethylformamide or a mixture thereof, and the semicrystalline cellulose nanofibers and/or cellulose nanocrystals. Most preferably, the solvent of the polar phase is water. The morphology, such as porosity of the shell wall of the capsule, which directly influences the oxygen barrier properties of the capsule, may be tailored by adding a coagulant medium soluble in the aqueous phase, preferably ethanol, methanol, acetone, isopropanol, dimethyl sulfoxides or dimethylformamide.

Preferably, in step b) no surfactant is added so that in step c) a Pickering emulsion is obtained, in which the cellulose nanofibers and/or cellulose nanocrystals stabilizes the emulsion. Good results are in particular obtained with a Pickering emulsion having an average surface charge density, which is the elementary charge per $nm^2$, of at most 0.07 $e/nm^2$, preferably at most 0.04 $e/nm^2$ and more preferably at most 0.035 $e/nm^2$.

According to an alternative embodiment, a surfactant and/or amphiphilic polysaccharide may be added to the polar phase and/or to the second phase, in order to form a stable dispersion in step c).

In principle, the cellulose nanofibers may be prepared from any cellulose material of natural origin or from synthetic cellulose. However, good results are in particular obtained with nanofibers being prepared from cellulose of natural origin. For example, the semicrystalline cellulose nanofibers may be prepared from plant cellulose, tunicin cellulose and/or bacterial cellulose e.g. by chemical pretreatment, acid hydrolysis, enzymatic treatment and/or mechanical disintegration.

A particular preferred example for a process for preparing the semicrystalline cellulose nanofibers comprises the steps of performing a mild acid hydrolysis of cellulose pulp, then homogenizing the hydrolysis product, thereafter performing a mild acid hydrolysis of the homogenized product and then dialyzing the hydrolysis product.

Moreover, the cellulose nanocrystals may be prepared from cellulose nanofibers by acid hydrolysis for example in 64 % by weight aqueous sulfuric acid.

Preferably, in step b) a mixture is provided, which contains a non-polar or hydrophobic solvent, a monomer, a catalyst and optionally at least one target compound to be encapsulated. In this case, in step c) an oil-in-water (O/W) dispersion is formed.

As non-polar or hydrophobic solvent any compound may be applied, which is able to dissolve the target compound, such as a solvent being selected from the group consisting of alkanes, alkenes, alkynes, aromatics, heterocyclics, ketones, aldehydes, triglycerides and arbitrary combinations of two or more of the aforementioned compounds.

All target compounds mentioned above for the nanocapsule, microcapsule and macrocapsule may be applied, such as in particular oils, fats, oils, colloids, micelles, food ingredients, drugs, dyes, phosphorescent compounds and photon upconversion compositions.

In principle, the present invention is not limited with regard to the monomer, oligomer and/or polymer added in step b). However, preferably the monomer, oligomer and/or polymer and the incubation conditions in step d) are so selected that a crosslinking of the cellulose nanofibers via the monomer, oligomer and/or polymer results. This crosslinking may be either achieved by covalent bonds generated during a chemical reaction between the cellulose nanofibers and/or cellulose nanocrystals and the monomer, oligomer and/or polymer, which is e.g. initiated by heat, a polymerization initiator, UV-light, an enzyme or a catalyst. Alternatively, an oligomer and/or polymer may be used, which adsorbs onto

the cellulose nanofiber and/or cellulose nanocrystal surface, thus crosslinking the cellulose nanofibers and/or cellulose nanocrystals by physical bonds or ionic bonds. Alternatively, such a physical bond may be accomplished by a solidification mechanism, namely for example by a solvent evaporation or a precipitation mechanism as a consequence of temperature, pH change, addition of non-solvent or salt during the preparation of the capsules. This may be e.g. accomplished by creating an O/W/O emulsion and evaporating the water under reduced pressure with the cellulose nanofibers being present in the polar phase. The O/W/O emulsion may be prepared by sonication, membrane techniques, T-junctions (microfluidics) or the like.

Suitable examples for the monomer are those selected from the group consisting of diisocyanate compounds, mixtures of diisocyanate compounds and diamines, mixtures of diisocyanate compounds and diols, acrylate compounds, methacrylate compounds, epoxides, lactames and mixtures of dicarboxylic acids and diamines, which lead after polymerization to polyurethanes, polyureas, polyacrylates, polymethacrylates, polyethers or polyamides, respectively. For example, the diisocyanate compound may be isophorone diisocyanate (IPDI) or toluene diisocyanate (TDI).

Suitable examples for the oligomer and/or polymer are hemicelluloses and their derivatives.

The dispersion may be effected in step c) by any process known to a person skilled in the art. Good results are in particular obtained, when the second phase is dispersed in the polar phase in step c) by ultrasonification. Alternatively, an equivalent high-speed mixing technique may be used.

The incubation conditions in step d), such as time and temperature, depend on the kind of monomer, oligomer and/or polymer applied in step a). In particular if a monomer is applied in step a), it is preferred that the incubation in step d) is performed at a temperature between 25 and 220°C for 1 minute to 5 days, in order to achieve a complete polymerization. Of course, the used temperature should consider the degradation temperature of the encapsulated compound

Finally, the obtained microcapsules may be dried, e.g. in vacuum at elevated temperature, in order to reduce its water content. The drying may be for example conducted at a temperature between 1 and 220°C at a pressure between and more than 0 and 100 kPa corresponding to 0.99 atm.

A further subject matter of the present invention is a capsule and in particular a nanocapsule, microcapsule or macrocapsule, which is obtainable with the aforementioned method.

In addition, the present invention relates to the use of a capsule and in particular a nanocapsule, microcapsule or macrocapsule described above for protecting a target compound against any selected from the groups consisting of gases, radicals, chemical compounds and physical evaporation, preferably against any selected from the groups consisting of ozone, oxygen prooxidants, radicals and peroxides and more preferably against oxygen, wherein the target compound is preferably selected from the group consisting of fats, oils, colloids, micelles, food ingredients, drugs, dyes and arbitrary combinations of two or more of the aforementioned compounds.

The capsule may be incorporated for its use into a film, foam or fiber. For example, films of various thickness containing the nanocapsules or microcapsules in a matrix of cellulose nanofibers or cellulose nanocrystals may be produced by solvent-casting, doctor-blading, spin-coating, electrospinning or wet-extrusion. By varying the weight ratio between the capsules and the matrix material it is possible to adjust the oxygen barrier properties to a desired value.

Preferably, the capsule in accordance with the present invention is used in a film, in a powder, in a foam, in a fiber, in an organic light-emitting device, in a solar cell, in an ink, in a thermal sensor, in an oxygen sensor, in a coating materials, in foodstuff, in a pharmaceutical composition or in a flexible display. More specifically, the nanocapsules, microcapsules or macrocapsules may be used to make stable functional food ingredients which can be sold and stored in dry state and having a long shelf life of several years. As further example, respective capsules containing photon upconversion dyes may be incorporated into cellulose nanofiber based films and used in this form in solar cell applications or in flexible displays for mobile phones or the like.

Potential further uses of the nanocapsule, microcapsule or macrocapsule may be systems for reporting the oxygen concentration or temperature based on photon upconversion principle using a combination of emitters and sensitizers. Such capsules could be injected in living organisms, where they report properties of the local environment.

Subsequently, the present invention is described in more detail by way of nonlimiting examples and comparative examples making reference to the figures, wherein:

- Fig. 1 is an atomic force microscope image of a blend of cellulose nanofibers and cellulose nanocrystals used in example 1.
- Fig. 2 is a scanning electron microscope image of nanocapsules obtained in example 1.
- Fig. 3 is a scanning electron microscope image showing the uniform thickness for the shell of the capsules obtained in example 1.
- Fig. 4 is an absorption spectrum of the sensitizer family used in example 1.
- Fig. 5 shows luminescence spectra of the TTA-UC composition used in example 1.
- Fig. 6 shows the dependency of the integral UC-fluorescence on the time for the TTA-UC composition used in example 1.

Example 1

A nanocapsule with a shell comprising a blend of semicrystalline cellulose nanofibers and semicrystalline cellulose nanocrystals (subsequently referred to as "NFC") has been prepared making use of never-dried bleached sulfite softwood cellulose pulp from spruce. The pulp contained 13.8 % by weight hemicellulose and 0.7 % by weight lignin. The semicrystalline cellulose nanofibers have been prepared from this pulp with the following three steps:

(1) Acid hydrolysis of sulfite pulp,
(2) high pressure homogenization and
(3) mild hydrolysis and subsequent dialysis.

In step (1) a 2.5 % by weight pulp fiber suspension (30 g dry) in 4 M HCl solution was heated at 80 ºC for 1.5 h. The hydrolyzed fibers were washed several times by diluting with MilliQ water and filtrating until pH of about 5. The product was redispersed in 2 L of MilliQ water and homogenized in step (2) by three passages through a high pressure homogenizer (M-110P, Microfludics, USA) at 1,650 bar and the chamber combination 400/100 μm. In step (3), concentrated HCl was added drop wise to the homogenized suspension to obtain 2.5 M HCl. The hydrolysis proceeded for 2 h at 90 ºC. The product was centrifugated (10,000 rpm) and the sediment was redispersed and dialyzed (MWCO 12-14 kDa) against MilliQ water until constant pH. The NFC suspension (dry content 1.71 wt%) was stored in the fridge. Prior to use, the NFC suspension was diluted and ultrasonicated (Branson Sonifier W-450-Digital with ½" tip). A NFC suspension having a concentration of 0.5 % by weight was prepared by ultrasonication for 120 s at 80% amplitude, followed by 60 s at 90% amplitude and under ice cooling. A 1 % by weight suspension was prepared by mixing a diluted 0.5 % by weight NFC suspension with stock suspension and ultrasonication at 80% amplitude for 120 s and then 90% amplitude for 120 s under ice cooling as polar NFC-phase.

An atomic force microscope image of the blend of cellulose nanofibers and cellulose nanocrystals obtained in this example is shown in Fig. 1. The nanofibers together with nanocrystals (around 140 in length) had an average length of 370 nm and an average diameter of 6.7 nm, as assessed from 270 entities. The sides of the image of Fig. 1 are 5.0 μm.

Moreover, a second oil phase containing 0.7 g hexadecane as solvent, 150 mg isophorone diisocyanate (IPDI) as crosslinker, dibutyltin dilaurate as catalyst in a molar ratio to IPDI of 1:6.5 and the following two sensitizers and the following emitter was prepared, wherein the total concentration of the two sensitizers in the oil phase was $1x10^{-4}$ mol/l and the concentration of the emitter in the oil phase was $1x10^{-3}$ mol/l.

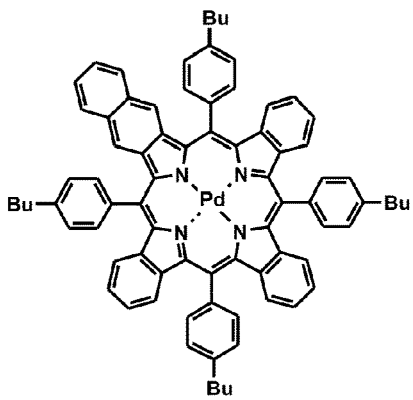

(1)

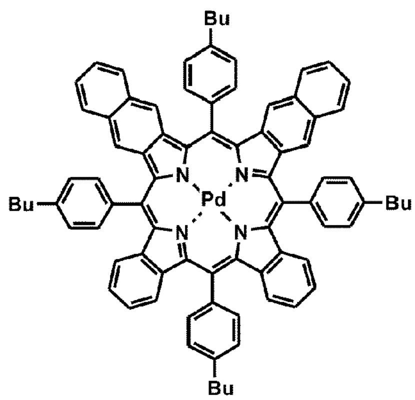

(2)

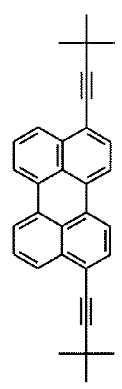

(3)

(1): 5,10,15,20-tetra(4-butylphenyl)naphtho-tribenzo-porphyrin palladium (II) (Pd1N3BP)
(2): 5,10,15,20-tetra(4-butylphenyl)-adj-dinaphtho-dibenzo-porphyrin palladium (II) (Pd2N2BP)
(3): 3,10-Bis(3,3-dimethylbut-1-ynyl)perylene (Y805).

Thereafter, 6.1 g of the 1 % by weight NFC-phase was mixed with the oil phase and the oil phase was dispersed in the NFC-phase by ultrasonication at 80% amplitude for 60 s under ice cooling. The emulsion was then placed in a heated oil bath at 40 ºC and left there under slow magnetic stirring overnight in order to effect a polyaddition reaction between the OH-groups of the cellulose material and the IPDI.

With this method, nanocapsules comprising a shell having an average thickness of about 30 nm and a core were obtained, wherein the core contained the sensitizers and the emitter dissolved in hexadecane, and wherein the shell comprised a blend of semicrystalline cellulose nanofibers and semicrystalline cellulose nanocrystals having each

a crystallinity index measured by X-ray diffraction of about 60%.

A Scanning electron microscope (SEM) image of the obtained capsules recorded with a Hitachi SU8000 scanning electron microscope is shown in Figure 2. The SEM image of the resulting liquid-core cellulose nanofiber-shell capsules clearly reveals the fibrous structure of the shell wall.

In Fig. 3 the uniform shell wall thickness of the resulting capsules is shown. The thickness of the shell wall was about 30 nm.

Then, an absorption spectrum of the sensitizers (1) and (2) in a molar ratio of 2:3 and luminescence spectra of the capsules at room temperature using an excitation light having a bandwidth of from 620 up to 680 nm and a varying excitation laser intensity of 0.1 to 5.8 W/cm$^2$ in an atmosphere having an oxygen concentration of 21 % were recorded. The results are shown in Fig. 4 to 6. As it derives from Fig. 4 to 6, the TTA-UC efficiencies for the capsules even oxygen saturated water suspensions are constant over the time showing that the capsules efficiently protect the TTA-UC composition from quenching by molecular oxygen. Taking into account, that 5 W/cm$^2$ could be obtained at more than 300 times the concentration of the sunlight for the broad bandwidth as shown in Figure 4, the proposed protection scheme will ensure stabile operation of upconverting solar devices.

## Claims

1. A capsule, preferably a nanocapsule, microcapsule or macrocapsule, comprising a shell and a core, wherein the core contains at least one compound being at least at any temperature between more than 0°C and 80°C at 101,325 kPa a liquid, and wherein the shell comprises semicrystalline cellulose nanofibers and/or semicrystalline cellulose nanocrystals having each a crystallinity index measured by X-ray diffraction of at least 30%.

2. The capsule in accordance with claim 1, wherein the shell comprises semicrystalline cellulose nanofibers and/or cellulose nanocrystals having a crystallinity index of at least 40%, preferably at least 50%, more preferably at least 55%, still more preferably of at least 60% and most preferably of at least 70%.

3. The capsule in accordance with claim 1 or 2, wherein the longest dimension of the shell is at most 1 cm, preferably between 0.1 and 5 μm, more preferably between 0.6 and 4 μm and most preferably between 1 and 2 μm.

4. The capsule in accordance with any of the preceding claims, wherein the cellulose nanofibers have an average length of between 1 and 5,000 nm, preferably between 1 and 1,000 nm, more preferably between 50 and 1,000 nm, even more preferably between 150 and 600 nm and most preferably between 250 and 500 nm, and/or, wherein the cellulose nanocrystals have an average length of between 40 and 2,000 nm, preferably between 60 and 2,000 nm, more preferably between 80 and 2,000 nm and most preferably between more than 100 and 2,000 nm.

5. The capsule in accordance with any of the preceding claims, wherein the cellulose nanofibers and/or cellulose nanocrystals have an average aspect ratio of between 5 and 1,000, preferably between 10 and 150, more preferably between 30 and 100 and most preferably between 50 and 80.

6. The capsule in accordance with any of the preceding claims, wherein the semicrystalline cellulose nanofibers and/or cellulose nanocrystals are in the form of a three-dimensional network, in which at least some of the semicrystalline cellulose nanofibers and/or cellulose nanocrystals are connected with one or more other semicrystalline cellulose nanofibers and/or cellulose nanocrystals of the shell of the capsule, preferably by any bond selected from covalent bonds, ionic bonds, physical bonds and combinations thereof.

7. The capsule in accordance with any of the preceding claims, wherein the shell further comprises an oligomer and/or a polymer, which is preferably selected from the group consisting of polyureas, polyurethanes, polyacrylates, polymethacrylates, polyethers, polyamides and arbitrary combinations of two or more of the aforementioned polymers.

8. The capsule in accordance with claim 7, wherein the weight ratio of semicrystalline cellulose nanofibers and/or cellulose nanocrystals to oligomer and/or polymer in the shell amounts to 100:0.1 to 0.1:99.9, preferably 80:20 to 10:90 and most preferably 70:30 to 14:86.

9. The capsule in accordance with any of the preceding claims, wherein the shell of the capsule has an elastic modulus of at least 1 GPa, preferably of at least 2 GPa, more preferably of at least 3 GPa, more preferably of at least 4.5 GPa, still more preferably of at least 10 GPa, still more preferably of at least 20 GPa and most preferably of at least

138 GPa.

**10.** The capsule in accordance with any of the preceding claims, wherein the core of the capsule contains as target compound any compound selected from the group consisting of fats, oils, colloids, drugs, food ingredients, micelles, dyes and arbitrary combinations of two or more of the aforementioned compounds.

**11.** The capsule in accordance with claim 10, wherein the target compound is at least one compound, which has a triplet state capable of energy transfer via an emissive process or a non-emissive process, and preferably is i) at least one phosphorescent compound or ii) a mixture of at least one sensitizer compound being capable of absorbing radiation at a first frequency $v_1$ and of at least one emissive compound, wherein the at least one sensitizer compound is capable of transferring energy to the at least one emissive compound and wherein the at least one emissive compound, after obtaining energy transferred from the at least one sensitizer compound, is capable of emitting light at a second frequency $v_2$, wherein the following equation is fulfilled: $v_2 > v_1$, wherein the at least one sensitizer compound is capable of a triplet-triplet energy transfer to the at least one emissive compound and wherein the at least one emissive compound is capable of a triplet-triplet annihilation

**12.** A method for preparing a capsule, preferably a nanocapsule, microcapsule or a macrocapsule, in accordance with any of the preceding claims comprising the steps of:

a) providing a polar phase containing semicrystalline cellulose nanofibers and/or cellulose nanocrystals having each a crystallinity index of at least 30%,
b) providing a second phase containing i) at least one solvent and/or dispersion agent being immiscible with the polar phase obtained in step a), ii) at least one compound selected from the group consisting of monomers, oligomers, polymers and arbitrary combinations thereof and optionally iii) at least one target compound to be encapsulated,
c) dispersing the second phase obtained in step b) in the polar phase obtained in step a) and
d) incubating the dispersion obtained in step c).

**13.** A capsule, preferably a nanocapsule, microcapsule or macrocapsule, which is obtainable with a method in accordance with claim 12.

**14.** Use of a capsule in accordance with any of claims 1 to 11 and 13 for protecting a target compound against any selected from the groups consisting of gases, radicals, chemical compounds and physical evaporation, preferably against any selected from the groups consisting of ozone, oxygen, prooxidants, radicals and peroxides and more preferably against oxygen, wherein the target compound is preferably selected from the group consisting of fats, oils, colloids, micelles, food ingredients, drugs, dyes and arbitrary combinations of two or more of the aforementioned compounds.

**15.** Use of a capsule in accordance with claim 14 in a film, in a powder, in a foam, in a fiber, in an organic light-emitting device, in a solar cell, in an ink, in a thermal sensor, in an oxygen sensor, in a coating materials, in foodstuff, in a pharmaceutical composition or in a flexible display.

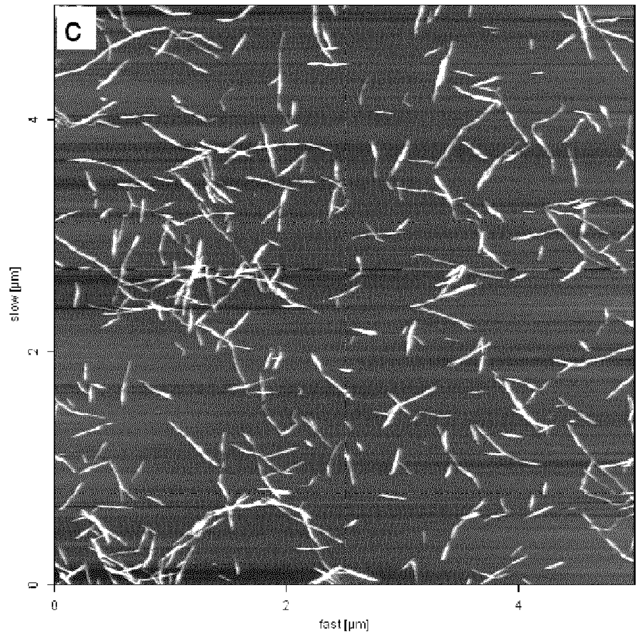


Fig. 1

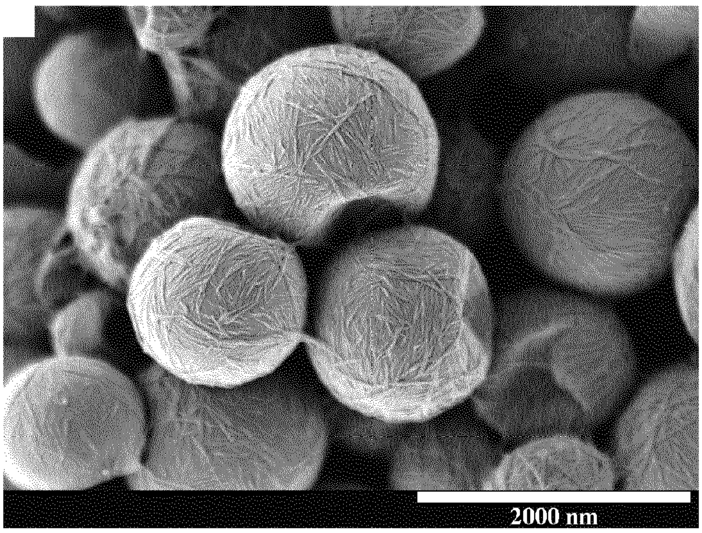


Fig. 2

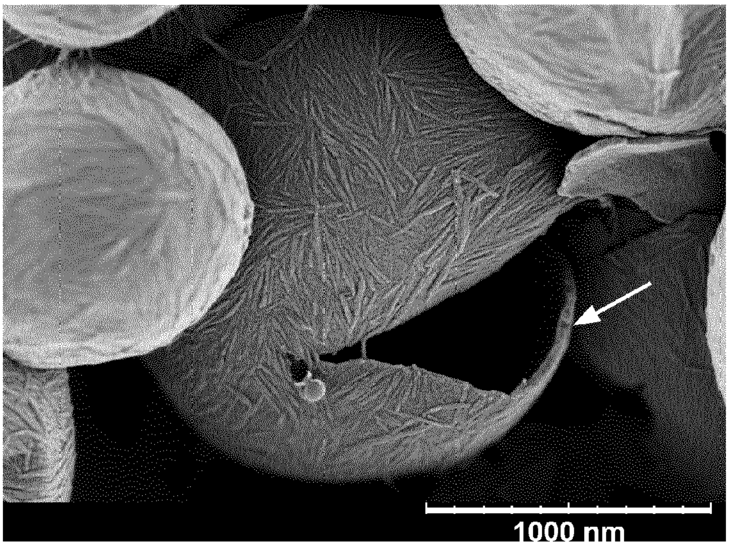
1000 nm

Fig. 3

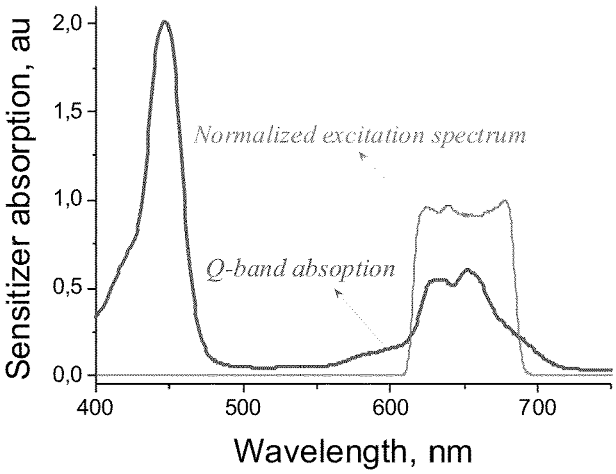
Sensitizer absorption, au
2,0
1,5
1,0
0,5
0,0
400
500
600
700
Wavelength, nm
Normalized excitation spectrum
Q-band absoption

Fig. 4

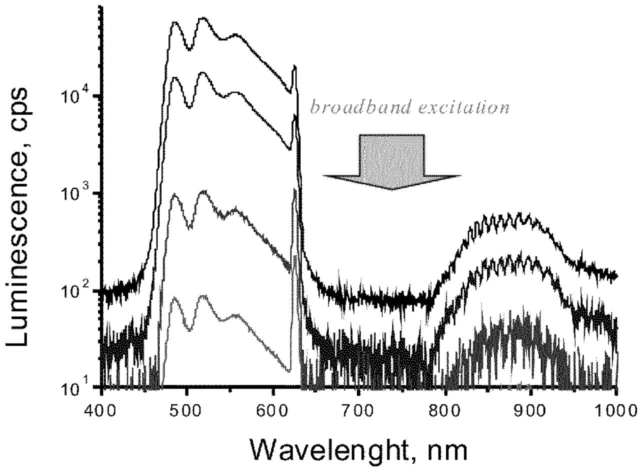
Luminescence, cps
$10^4$
$10^3$
$10^2$
$10^1$
400
500
600
700
800
900
1000
Wavelenght, nm
broadband excitation

Fig. 5

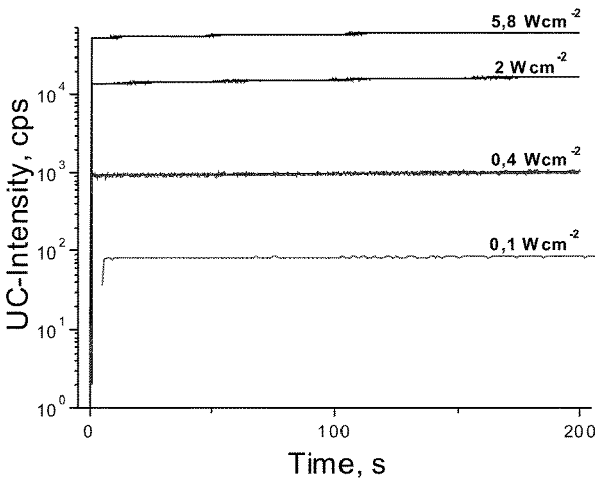
UC-Intensity, cps
$10^4$
$10^3$
$10^2$
$10^1$
$10^0$
0
100
200
Time, s
5,8 Wcm-2
2 Wcm-2
0,4 Wcm-2
0,1 Wcm-2

Fig. 6

Europäisches Patentamt
European Patent Office
Office européen des brevets

# EUROPEAN SEARCH REPORT

Application Number
EP 13 19 0091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PING LU ET AL: "Cellulose isolation and coreshell nanostructures of cellulose nanocrystals from chardonnay grape skins", CARBOHYDRATE POLYMERS, APPLIED SCIENCE PUBLISHERS, LTD. BARKING, GB, vol. 87, no. 4, 8 November 2011 (2011-11-08), pages 2546-2553, XP028349359, ISSN: 0144-8617, DOI: 10.1016/J.CARBPOL.2011.11.023 [retrieved on 2011-11-17] * page 2550 - page 2552 * * figure 8(b) * ----- | 1-15 | INV. B01J13/12 B01J13/14 A23P1/04 A61K9/50 C09B67/02 |
| A | DATABASE WPI Week 197721 Thomson Scientific, London, GB; AN 1977-36915Y XP002720947, & JP S52 45589 A (TOYO JOZO KK) 11 April 1977 (1977-04-11) * the whole document * ----- | 1-15 | |
| A | WO 2010/079468 A2 (PROCTER & GAMBLE [US]; DIHORA JITEN ODHAVJI [US]; SMETS JOHAN [BE]; SC) 15 July 2010 (2010-07-15) * the whole document * ----- | 1-11, 13-15 | TECHNICAL FIELDS SEARCHED (IPC) B01J A23P A61K C09B |
| A | DATABASE WPI Week 199933 Thomson Scientific, London, GB; AN 1999-389395 XP002720948, & JP H11 152233 A (ASAHI KASEI KK) 8 June 1999 (1999-06-08) * the whole document * ----- | 1-11,13 | |

The present search report has been drawn up for all claims

1

| Place of search | Date of completion of the search | Examiner |
|---|---|---|
| The Hague | 27 February 2014 | Tarallo, Anthony |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 0091

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2014

| Patent document cited in search report | Publication date | Patent family member(s) | Publication date |
|---|---|---|---|
| JP S5245589 A | 11-04-1977 | JP S5245589 A | 11-04-1977 |
| | | JP S5524938 B2 | 02-07-1980 |
| WO 2010079468 A2 | 15-07-2010 | CA 2795617 A1 | 15-07-2010 |
| | | CN 102892492 A | 23-01-2013 |
| | | EP 2563508 A2 | 06-03-2013 |
| | | JP 2013525564 A | 20-06-2013 |
| | | KR 20130000417 A | 02-01-2013 |
| | | US 2011268802 A1 | 03-11-2011 |
| | | WO 2010079468 A2 | 15-07-2010 |
| JP H11152233 A | 08-06-1999 | JP 4260237 B2 | 30-04-2009 |
| | | JP H11152233 A | 08-06-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Encapsulated triplet-triplet annihilation-based upconversion in the aqueous phase for sub-band-gap semiconductor photocatalysis. *Journal of the American Chemical Society,* 2012, vol. 134, 17478-17481
- **LIU et al.** A general strategy for biocompatible, high-effective upconversion nanocapsules based on triplet-triplet annihilation. *Journal of the American Chemical Society,* 2013, vol. 135, 5029-5037
- **PARK et al.** Cellulose crystallinity index: measurement techniques and their impact on interpreting cellulose performance. *Biotechnology for Biofuels,* 2010, vol. 3, 10
- **SEGAL et al.** An empirical method for estimating the degree of crystallinity of native cellulose using the x-ray diffractometer. *Text Res J,* 1959, 786-794
- **A. FERY ; R. WEINKAMER.** *Polymer,* 2007, vol. 48, 7221-7235
- **H. J. BUTT ; B. CAPPELLA ; M. KAPPL.** *Surf Sci Rep,* 2005, vol. 59, 1-152